# EUROPEAN PATENT SPECIFICATION

(11) **EP 0 378 059 B1**
(45) Date of publication and mention of the grant of the patent: **22.09.1993**
(21) Application number: 89850434.5
(22) Date of filing: 15.12.1989
(51) Int. Cl.: G01N 33/543, G01N 33/534, G01N 33/533, G01N 33/542

(54) **Fibrous support for scintillation proximity assays**
Faserstoffträger für Scintillationsannäherungsmesssysteme
Support fibreux pour des épreuves de scintillation de proximité

(30) Priority: 27.12.1988 SE 8804657
(43) Date of publication of application: 18.07.1990
(73) Proprietor: Potter, Colin Gerald, Headington Oxford OX3 7RR (GB); Warner, Gerald Truscott, Headington Oxford (GB)
(72) Inventor: Potter, Colin Gerald, Headington Oxford OX3 7RR (GB); Warner, Gerald Truscott, Headington Oxford (GB)
(74) Representative: Engholm, Carl

(56) References cited:
- EP-A- 0 154 734
- GB-A- 2 007 362

## Description

The invention relates to a support structure for use in a proximity assay to detect a radiolabelled reactant in a liquid sample.

U.S. Patent No. 4,568,649 describes a proximity assay whereby binding molecules, usually immunoglobulins, are themselves bound to the surface of scintillant support particles. The particles may be in the form of small beads. A solution containing radioactive ligand of known activity, together with a sample containing an unknown quantity of unlabelled ligand are incubated with the beads. If the labelling isotope has a low energy emission, for example tritium, then only that portion of the sample that binds to the immunoglobulin and is in close proximity (a few µm) to the scintillant, will result in scintillation events that can be counted on a liquid scintillation counter, unbound molecules being at too great a distance to register. After incubation, the competition of labelled and unlabelled ligand, will bring different proportions of labelled ligand in close proximity to the scintillant. This technique does not require physical separation of the bound component from the remnant of the sample. However, since the beads are suspended in a liquid medium, this technique is prone to spillage and requires large volumes of assay medium to be used in scintillation vials for counting in standard scintillation counters.

The object of the invention is to improve the support structures used so far in proximity assays. This is attained in that the support structure according to the invention is a fibre mat having a fluorescer incorporated therewith and a ligand attached thereto, said ligand being capable of binding said reactant to activate the fluorescer to produce photons to be detected.

According to the invention, the support structure is a fibre mat which in a first embodiment comprises solid scintillant fibres forming a matrix. The solid scintillant may be a glass, for example cerium-loaded glass or based on rare earths such as yttrium silicate which produce efficient scintillators. Such glasses may also incorporate activators such as terbium, europium or lithium. Alternatively the fibre mat may be made from a scintillant loaded polymer such as polyvinyltoluene.

As an alternative to scintillant fibres, the scintillant may be coated onto a fibre mat of non-scintillant fibres by evaporation of a solvent containing the scintillant. Suitable scintillants include PPO (2,5-diphenyloxazole), anthracene, Butyl-PBD [2-(4'-tert-butylphenyl)-5(4''-biphenyl)-1,3,4-oxadiazole] or PMP (1-phenyl-3-mesityl-2-pyrazoline) together with or without spectral shifters such as POPOP ([1,4-di-(2-(5-phenyloxazolyl)-benzine] or bis-MSB [1,4-di(2-methylsteryl)-benzine]. A solid phase material which acts as a solid solvent may be used, for example tetramethylbenzine. Solid scintillants that are hydrophobic may be modified by surfactants to accept aqueous solutions.

Alternatively, scintillant material may be introduced during manufacture of the fibrous matrix by coating with water soluble scintillants. These can be made from normally hydrophobic organic scintillants by the process of sulphonation to produce their sodium salts. If required, insolubility in water can be restored by end-capping, produced for example by silanization.

The different ligands that can be measured by the described structure include protein antigens such as alphafetoprotein, carcinoembryonic antigen or enzymes such as creatine kinases and prostatic acid phosphatase. Micro-organisms such as viruses, bacteria, fungi and parasites in a wide range of species and varieties may be assayed. These examples will, in general be measured by immunoglobulins attached to the fibrous scintillant-matrix but other binding molecules may also be used such as nucleic acids or nucleic-acid-binding moieties. Complementary sequences of nucleic acid may be used in this way to detect a wide range of specific nucleic acid sequences. For example, bacteria and other species can be detected to almost any level of specificity by chosing a suitable ribosomal-RNA sequence. In a different aspect, a rare DNA sequence, made plentiful by the polymerase chain reaction amplification technique may be detected in this assay. Naturally, assay materials may be made by conventional extraction procedures or directly from body fluids.

The fibres of the described solid scintillant matrix present a large surface area for binding, but in addition it is a feature of the present invention that capilliary action can be used to permeate the matrix with the sample and once filled will hold the sample within the matrix for subsequent counting, after the filter bearing the sample or multiple samples is enclosed and sealed in a container such as a plastic bag. The flat format proposed here both retains the sample and permits good counting geometry for flat bed scintillation counters of the type described in U.S. Patent No. 4,298,796. The small volume required results in low volumes of radioactive waste which are in a more secure form than liquids in multiple wells for disposal. It also reduces the incubation time required for the assay.

The matrix plus binding molecules can bear multiple samples directly, or the positions and patterns of deposition of the samples may be constrained by permeation of an impermeable barrier around the sample area such as latex or synthetic resin thus leaving separated samples but on a physically continuous support. In that case the barriers are hydrophobic while the sample areas are hydrophilic. The fibre mat may exhibit more than one assay area, each said assay area being delineated by a barrier extending through the thickness of the fibre mat.

Alternatively the fibre mat is in the form of a small disc. The small discs may be fixed in a sealable well of a thin transparent or translucent tray. The discs of the scintillant matrix, bearing the immunoglobulins may be affixed to shallow wells of a supporting tray. The discs can be made by punching out from uniform sheets of the material and it is a feature of this invention that it is possible to produce discs which are highly reproducible in weight which permits accurate dispensing of the amount of binding molecule on which the accuracy of the assay depends. Furthermore, delineation of sample areas by any of the barriers can also be used to determine the amount of binding molecule presented to the mixture of sample and radioactive ligand.

## Claims

1. A support structure for use in a proximity assay to detect a radiolabelled reactant in a liquid sample, characterized in that the support structure is a fibre mat having a fluorescer incorporated therewith and a ligand attached thereto, said ligand being capable of binding said reactant to activate the fluorescer to produce photons to be detected.

2. Structure according to claim 1, characterized in that the fibre mat is in the form of a small disc.

3. Structure according to claim 2, characterized in that the small disc is fixed in a sealable well of a thin transparent or translucent tray.

4. Structure according to claim 1, characterized in that the fibre mat exhibits more than one assay area, each said assay area being delineated by a barrier extending through the thickness of the fibre mat.

5. Structure according to claim 4, characterized in that the barrier is hydrophobic.

## Patentansprüche

1. Trägerstruktur zur Verwendung in einem Annäherungstest zum Nachweis eines radioaktiv markierten Reaktanten in einer flüssigen Probe, dadurch gekennzeichnet, daß die Trägerstruktur ein Faservlies ist, in das eine fluoreszierende Substanz eingearbeitet ist, und an das ein Ligand gebunden ist, wobei der Ligand den Reaktanten unter Aktivierung der fluoreszierenden Substanz zur Erzeugung von nachzuweisenden Photonen binden kann.

2. Struktur nach Anspruch 1, dadurch gekennzeichnet, daß das Faservlies in Form einer kleinen Scheibe vorliegt.

3. Struktur nach Anspruch 2, dadurch gekennzeichnet, daß die kleine Scheibe in einer versiegelbaren Kavität eines dünnen, durchsichtigen oder durchscheinenden Sammelbehältnisses fixiert ist.

4. Struktur nach Anspruch 1, dadurch gekennzeichnet, daß das Faservlies mehr als eine Testfläche besitzt, wobei jede Testfläche von einer Barriere, die sich durch die Dicke des Faservlieses erstreckt, umgrenzt ist.

5. Struktur nach Anspruch 4, dadurch gekennzeichnet, daß die Barriere hydrophob ist.

## Revendications

1. Structure de support destinée à être utilisée dans une analyse de proximité pour détecter un corps réactionnel radiomarqué dans un échantillon liquide, caractérisée en ce que la structure de support est un mat de fibres auquel est incorporé un agent engendrant une fluorescence et auquel est fixé un ligand, ledit ligand étant capable de fixer ledit corps réactionnel pour activer l'agent engendrant une fluorescence afin de produire des photons à détecter.

2. Structure suivant la revendication 1, caractérisée en ce que le mat de fibres est sous forme d'un petit disque.

3. Structure suivant la revendication 2, caractérisée en ce que le petit disque est fixé dans un puits, pouvant être obturé de manière étanche, d'un plateau transparent ou translucide mince.

4. Structure suivant la revendication 1, caractérisée en ce que le mat de fibres présente plus d'une surface d'analyse, chacune desdites surfaces d'analyse étant délimitée par une barrière s'étendant à travers l'épaisseur du mat de fibres.

5. Structure suivant la revendication 4, caractérisée en ce que la barrière est hydrophobe.
